# EUROPÄISCHE PATENTSCHRIFT

(11) **EP 1 981 403 B1**
(45) Veröffentlichungstag und Bekanntmachung des Hinweises auf die Patenterteilung: **02.04.2014**
(21) Anmeldenummer: 07721869.1
(22) Anmeldetag: 23.01.2007
(51) Int. Cl.: A61B 5/11, A61C 19/045

(54) **VERFAHREN UND VORRICHTUNG ZUR AUFZEICHNUNG VON KÖRPERBEWEGUNGEN**
METHOD AND DEVICE FOR THE RECORDING OF BODY MOVEMENTS
PROCEDE ET DISPOSITIF POUR ENREGISTRER LES MOUVEMENTS D'UN CORPS

(30) Priorität: 26.01.2006 DE 102006003945; 27.01.2006 DE 102006004197
(43) Veröffentlichungstag der Anmeldung: 22.10.2008
(73) Patentinhaber: Dental Innovation GmbH, 44227 Dortmund (DE)
(72) Erfinder: Klett, Rolf, 97204 Höchberg (DE)
(74) Vertreter: Castell, Klaus
(86) Internationale Anmeldenummer: PCT/DE2007/000146
(87) Internationale Veröffentlichungsnummer: WO 2007/085241

(56) Entgegenhaltungen:
- WO-A-2004/023783
- DE-A1- 10 339 241

## Beschreibung

Die Erfindung betrifft ein Verfahren zur Aufzeichnung von Position, Lage oder Bewegung eines Körperteils eines Patienten mit Hilfe elektronischer Bilderkennung gemäß Patentanspruch 1, sowie eine Vorrichtung zur Aufzeichnung von Position, Lage oder Bewegung eines Körperteils eines Patienten oder eines ärztlichen Instruments gemäß dem Oberbegriff des Patentanspruchs 21.

Verfahren zur Aufzeichnung der Position, der räumlichen Lage oder von Bewegungsbahnen zumindest von Teilen des menschlichen Körpers oder allgemein von Körperteilen von Patienten, bzw. dementsprechende Vorrichtungen, kommen in verschiedensten Bereichen, ausgehend beispielsweise von Wissenschaft und Forschung, über den Medizinbereich bis hin zur Medienproduktion zum Einsatz, und sind z.B. aus der WO 2004/023783 oder DE 103 39 241 bekannt.

So ist es zum Beispiel bekannt, Position, Lage oder Bewegung von Körpergliedern mittels mechanischer bzw. elektromechanischer Abnehmer aufzuzeichnen und die gewonnenen Daten auf vorzugsweise elektronische Prozessor- und Speichermedien zu übertragen. Auf diese Weise lassen sich beispielsweise Bewegungsauswertungen im Rahmen der Trainingsbetreuung von Sportlern erstellen, es lassen sich anhand der gewonnenen Positions- und Bewegungsdaten mechanische Aktuatoren wie Roboterarme steuern oder digitale Visualisierungen im Film- und Medienbereich erstellen, oder die gewonnenen Daten werden beispielsweise zur Unterstützung bei der medizinischen Diagnose oder Therapie verwendet.

Außer den mechanischen bzw. elelctromechanischen Abnehmern zur Ermittlung von Position, Lage oder Bewegung von Körpergliedern bzw. Körperteilen sind weiterhin auch berührungslos, beispielsweise optisch oder auch mit Ultraschall und dgl. arbeitende Abnehmersysteme bekannt. Bei derartigen bekannten berührungslosen Abnehmersystemen werden die auszuwertenden Körperteile im Allgemeinen zunächst einmal mit leicht zu verfolgenden Reflektoren oder Leuchtpunkten, wie beispielsweise mit aufgeklebten Leuchtdioden oder dergleichen, versehen. Anschließend wird der zu untersuchende Bewegungsablauf aufgezeichnet, indem eine Sensoreinrichtung bzw. Bildaufnahmekamera auf die mit den Leuchtpunkten versehenen Körperteile gerichtet wird und sodann entsprechende Film- bzw. Bildaufnahmen der Körperteile bzw. der Leuchtpunkte erstellt werden.

Auch sind im medizinischen Bereich Vorrichtungen zur Erfassung der Position bzw. zur Navigation von ärztlichen Instrumenten, beispielsweise von chirurgischen Instrumenten bekannt, bei denen die Position oder Bewegungsbahn zumindest von Teilen des ärztlichen Instruments auf ähnliche Weise verfolgt wird. Hierdurch kann einem Chirurgen, beispielsweise mit Hilfe eines manuell gesteuerten Manipulators, ein präziseres Arbeiten als per Hand ermöglicht werden, da durch den Manipulator das ärztliche Instrument aufgrund der Positionsverfolgung sowie aufgrund einer Bewegungsübersetzung präziser geführt werden kann und Ungenauigkeiten oder Zittern des Chirurgen auf diese Weise kompensiert werden können. Ebenso werden derartige Vorrichtungen zur Erfassung der Position bzw. zur Navigation von ärztlichen Instrumenten bei der sog. Robotermedizin eingesetzt, beispielsweise bei der automatisch gesteuerten Ausfräsung des Oberschenkelhalses zum Zweck der nachfolgenden Verankerung einer Hüftgelenk-Endoprothese.

Derartige bekannte Verfahren bzw. Vorrichtungen zur Aufzeichnung von Position, Lage oder Bewegung von Körperteilen von Patienten bzw. Vorrichtungen zur Bewegungsverfolgung von ärztlichen Instrumenten sind jedoch einerseits zumeist aufwändig und erlauben zudem häufig allenfalls eine mittlere Genauigkeit bezüglich der Ermittlung der Position bzw. der räumlichen Lage bei der Aufzeichnung. Zu einer Erhöhung der Genauigkeit ist es bei den bekannten Verfahren bzw. Vorrichtungen durchweg notwendig, die Relativposition zwischen dem verfolgten Körperteil oder Instrument und der verfolgenden Sensorik aufwändig zu kalibrieren, um auf diese Weise die gewünschten exakten quantitativen Aussagen über den Ort bzw. die Bewegungskurven des verfolgten Körperteils oder Instruments treffen zu können, wobei zudem bei jeder größeren Veränderung zwischen dem verfolgten Körperteil oder Instrument und der verfolgenden Sensorik eine erneute Kalibrierung notwendig ist, um Messfehler auszuschließen.

Mit diesem Hintergrund ist es die Aufgabe der vorliegenden Erfindung, ein Verfahren zur Aufzeichnung von Position, Lage oder Bewegung von Körperteilen und eine Vorrichtung zur Positions- bzw. Bewegungsverfolgung von Körperteilen oder ärztlichen Instrumenten zu schaffen, womit sich die genannten, beim Stand der Technik vorhandenen Nachteile überwinden lassen sollen. Insbesondere soll dabei sowohl ein hoher Grad an Automatisierung als auch eine sehr hohe Genauigkeit der Positionsermittlung erreicht werden; das Verfahren und die Vorrichtung sollen ferner mit besonders geringem apparativem Aufwand bei gleichzeitig besonders großer Flexibilität einsetzbar sein, und die Rückwirkung auf das zu messende Objekt oder Körperteil bzw. auf den Patienten durch die Aufzeichnung bzw. Messung soll minimiert werden.

Diese Aufgabe wird durch ein Verfahren gemäß Patentanspruch 1 bzw. durch eine Vorrichtung mit den Merkmalen des Patentanspruchs 21 gelöst.

Bevorzugte Ausführungsformen sind Gegenstand der Unteransprüche.

Das Verfahren gemäß der Erfindung dient zur Aufzeichnung von Position, Lage oder Bewegung zumindest eines Teils eines Körpers eines Patienten. Dabei wird der Einfachheit und Einheitlichkeit halber durchweg der Begriff "Patient" verwendet, wobei hierdurch jedoch keineswegs eine Einschränkung der Erfindung etwa auf medizinische Bereiche impliziert sein soll. Vielmehr ist der Begriff "Patient" im Kontext der vorliegenden Erfindung im weitesten Sinne als Synonym für "zu beobachtendes Lebewesen" zu verstehen.

Dabei ist der zumindest eine Teil des Körpers des Patienten zunächst einmal in einem Objektraum angeordnet und lässt sich mit Hilfe zumindest einer Bildaufnahmekamera in einen Bildraum abbilden. Die Unterscheidung in "Objektraum" und "Bildraum" wird vorliegend getroffen, um begrifflich eine klare Unterscheidung zwischen den geometrischen Zusammenhängen am Objekt bzw. am Patienten einerseits (Objektraum) und den geometrischen Zusammenhängen innerhalb des von der Bildaufnahmekamera erzeugten Bildes (Bildraum) treffen zu können.

Im Rahmen des erfindungsgemäßen Verfahrens wird dabei in einem ersten Verfahrenschritt a) zumindest eine Grafikmarkierung mit zumindest einem Körperteil des Patienten verbunden. Dabei ist zumindest ein geometrisches Größenbeschreibungsmerkmal der Grafikmarkierung bekannt. Als lediglich erläuterndes, keineswegs jedoch beschränkendes Beispiel für eine derartige, mit dem Körperteil des Patienten zu verbindende Grafikmarkierung sei im einfachsten Fall eine kreisförmige, aufgeklebte Marke genannt, wobei im Fall der einfachen kreisförmigen Marke das Größenbeschreibungsmerkmal in dem bekannten Durchmesser der kreisförmigen Marke im Objektraum besteht.

In einem weiteren Verfahrenschritt b) wird sodann eine Abbildung zumindest des die Grafikmarkierung enthaltenden Bereichs des Körperteils durch die Bildaufnahmekamera erstellt.

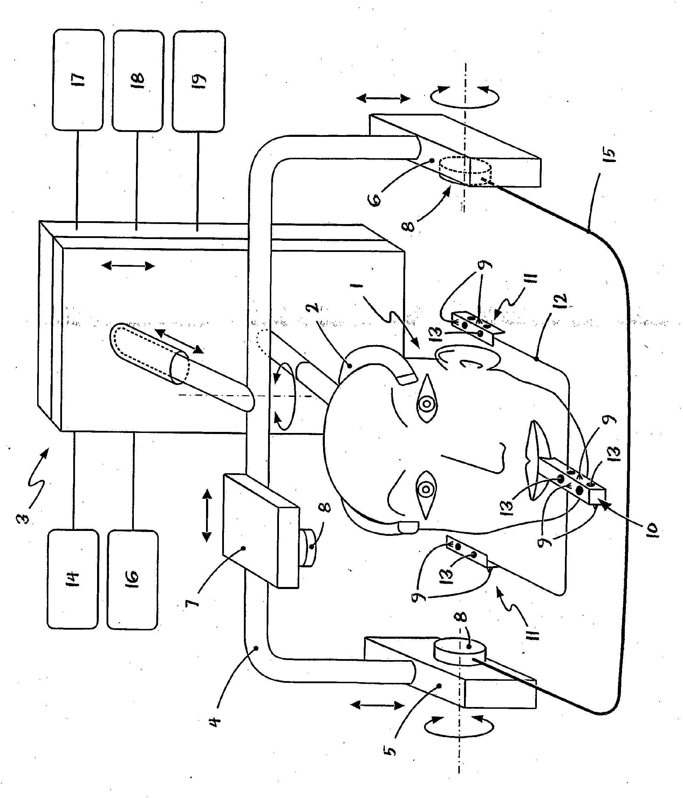

Dann erfolgt in einem weiteren Verfahrenschritt c) eine Übernahme der Abbildung als Bilddatei, beispielsweise als Pixelbild, aus der Bildaufnahmekamera in eine elektronische Speicher- bzw. Prozessoreinrichtung.

In einem weiteren Verfahrenschritt d) erfolgt sodann innerhalb des Bildraums der Bilddatei eine automatische Lokalisierung des in der Bilddatei enthaltenen Abbilds der Grafikmarkierung mittels Anwendung einer Bilderkennungsprozedur durch die Prozessoreinrichtung auf die Bilddatei.

Während eines weiteren Verfahrensschritts e) wird sodann das Größenbeschreibungsmerkmal der Grafikmarkierung im Bildraum ermittelt. Dies bedeutet im oben genannten Beispiel der als Grafikmarkierung verwendeten einfachen kreisförmigen Marke, dass durch die Prozessoreinrichtung in diesem Verfahrensschritt beispielsweise ermittelt wird, wie vielen aneinander gereihten Bildpixeln (falls die Bilddatei beispielsweise in Form eines Pixelbilds vorliegt) der Durchmesser der Abbildung der kreisförmigen Marke in der Bilddatei entspricht.

In einem weiteren Verfahrensschritt f) wird sodann die Position und/oder die Lage der Grafikmarkierung im Objektraum relativ zur Bildaufnahmekamera ermittelt. Dies erfolgt anhand des nun sowohl im Objektraum als auch im Bildraum bekannten Größenbeschreibungsmerkmals der Grafikmarkierung. In diesem Verfahrensschritt erfolgt mit anderen Worten eine automatische Kalibrierung der Bildaufnahmekamera jeweils auf die vorliegenden geometrischen Relationen zwischen dem Ort der Bildaufnahmekamera und dem Ort des Körperteils bzw. dem Ort der darauf angeordneten Grafikmarkierung.

Denn aus dem sowohl im Objektraum als auch im Bildraum bekannten Größenbeschreibungsmerkmal (Beispiel: kreisförmige Marke hat den bekannten Durchmesser "20 mm" im Objektraum und gleichzeitig den ermittelten Durchmesser "328 Pixel" im Bildraum) kann unter Berücksichtigung der Abbildungseigenschaften des Kameraobjektivs sowohl der tatsächliche Abstand zwischen Grafikmarkierung und Bildaufnahmekamera als auch der Abstand zwischen der Grafikmarkierung und der optischen Achse der Bildaufnahmekamera ermittelt werden. Überdies kann auch noch eine etwaige Winkelkippung der Grafikmarkierung gegenüber der optischen Achse der Bildaufnahmekamera ermittelt werden. Im Beispiel der einfachen kreisförmigen Marke kann dies anhand der ohne weiteres möglichen Feststellung der beiden Halbachsen des im Bildraum im Allgemeinen elliptischen Abbilds der kreisförmigen Marke erfolgen.

Das erfindungsgemäße Verfahren erlaubt somit die Ermittlung sowohl der Position im dreidimensionalen Raum als auch die Feststellung der Winkellage des Körperteils, bzw. der mit dem Körperteil verbundenen Grafikmarkierung, relativ zur Bildaufnahmekamera, ohne dass dabei die Notwendigkeit einer manuellen Kalibrierung der Kamera gegeben wäre. Damit wird aber zunächst einmal bereits eine Vielzahl von möglichen Anwendungen erschlossen bzw. gegenüber den aus dem Stand der Technik bekannten Verfahren maßgeblich erleichtert und verbessert. So lässt sich mit dem erfindungsgemäßen Verfahren beispielsweise ein mit einer Grafikmarkierung versehener Körperteil eines Patienten im dreidimensionalen Obj ektraum sowohl bezüglich seiner räumlichen Position als auch bezüglich seiner Winkellage lokalisieren, und zwar weitgehend unabhängig von Ort und Platzierung der Bildaufnahmekamera sowie ohne Notwendigkeit der Kamerakalibrierung vor Beginn der Messungen.

Dabei wird die Genauigkeit der Lokalisierung der Grafikmarkierung bzw. des beobachteten Objekts gegenüber dem Stand der Technik insbesondere bereits dadurch erheblich verbessert, dass das erfindungsgemäße Verfahren nicht nur Aussagen über die Position bzw. Auslenkung der Grafikmarkierung gegenüber der optischen Achse der Bildaufnahmekamera zulässt, sondern zudem den Abstand zwischen Objekt bzw. Grafikmarkierung und Bildaufnahmekamera liefert. Anhand des damit stets bekannten Abstands zwischen Objekt und Bildaufnahmekamera können aber nicht mehr nur qualitative oder näherungsweise Aussagen über die Position des Objekts getroffen werden, sondern Ort und Lage des Objekts können im dreidimensionalen Raum quantitativ mit hoher Genauigkeit bestimmt werden.

Mit dem Hintergrund auch der Ermittlung von Bewegungsabläufen ist es gemäß einer besonders bevorzugten Ausführungsform des erfindungsgemäßen Verfahrens ferner vorgesehen, dass zur Aufzeichnung einer Ortsveränderung der Grafikmarkierung bzw. des Körperteils die Verfahrensschritte b) bis g) wiederholt durchlaufen werden. Auf diese Weise werden aufeinander folgende Abbildungen des die Grafikmarkierung enthaltenden Bereichs des Körperteils erzeugt, anhand deren sukzessiver Auswertung durch die Prozessoreinrichtung Bewegungsabläufe bzw. Bewegungskurven der Grafikmarkierung bzw. des Körperteils erstellt werden können. ,

Gemäß einer weiteren Ausführungsform des erfindungsgemäßen Verfahrens ist im Falle von zumindest zwei relativbeweglichen Körperteilen jeweils zumindest eine Grafikmarkierung mit jedem der relativbeweglichen Körperteile verbunden. Auf diese Weise lässt sich die Relativposition der betreffenden Körperteile bzw. lassen sich Relativstellungen und Relativbewegungen zwischen den Körperteilen mit hoher Zuverlässigkeit und Genauigkeit bestimmen und verfolgen. Nach einer weiteren Ausführungsform ist hingegen vorgesehen, dass mit ein und demselben Körperteil zumindest zwei Grafikmarkierungen verbunden sind. Auf diese Weise lässt sich die Position des Körperteils wie auch die Lage des Körperteils im dreidimensionalen Raum mit besonders hoher Genauigkeit bestimmen. Letzteres gilt insbesondere dann, wenn auch eine bekannte geometrische Relation, beispielsweise der bekannte Abstand zwischen den zumindest zwei Grafikmarkierungen, in die Auswertung durch die Prozessoreinrichtung mit einbezogen wird.

Gemäß weiterer Ausführungsformen des erfindungsgemäßen Verfahrens sind zumindest zwei Bildaufnahmekameras vorgesehen. Dabei erfolgt gemäß einer Ausführungsform die Aufnahme ein und derselben Grafikmarkierung durch die zumindest zwei Bildaufnahmekameras. Auf diese Weise lässt sich die Zuverlässigkeit und Genauigkeit der Ermittlung der Position bzw. Lage der Grafikmarkierung im dreidimensionalen Objektraum weiter steigern.

Gemäß einer weiteren Ausführungsform sind jedoch mit zumindest einem Körperteil zumindest zwei Grafikmarkierungen verbunden, wobei die Grafikmarkierungen jeweils unterschiedlichen, vorzugsweise kartesischen bzw. orthogonalen Raumrichtungen zugeordnet sind, und wobei für jede der Raumrichtungen eine eigene Bildaufnahmekamera vorgesehen ist. Auf diese Weise kann die Ermittlung der Position bzw. Winkellage oder die Aufzeichnung eines Bewegungsablaufs des Körperteils im dreidimensionalen Raum mit noch weiter erhöhter Genauigkeit erfolgen.

Wie bereits eingangs dargestellt, eignet sich das erfindungsgemäße Verfahren für unterschiedlichste Anwendungsbereiche, bei denen das Wissen über Absolutposition, Relativposition oder über Bewegungsabläufe von Körperteilen von Interesse ist. Gemäß besonders bevorzugter Ausführungsformen des erfindungsgemäßen Verfahrens sind die ermittelten Positionsdaten jedoch für orthopädische oder zahntechnische Zwecke, beispielsweise zur genauen Registrierung von Kieferbewegungen, vorgesehen. Die Erfindung lässt sich besonders vorteilhaft in diesen Bereichen einsetzen, insbesondere insofern, als die hierzu bisher verwendeten, zumeist elektromechanischen Verfahren und Aufzeichnungsgeräte häufig mit äußerst hohem apparativem Aufwand sowie mit dementsprechend hohen Kosten wie auch mit vergleichsweise hoher patientenseitiger Belastung verbunden sind.

Die Erfindung lässt sich ferner auch unabhängig davon verwirklichen, wie die verwendete Grafikmarkierung bzw. die Mehrzahl am Grafikmarkierungen konkret ausgestaltet ist bzw. welche bekannten geometrischen Beschreibungsmerkmale die Grafikmarkierung aufweist, solange zumindest ein genügender Kontrast vorhanden ist, der die Erfassung und automatische Auswertung der Aufnahmen aus der Bildaufnahmekamera durch die Prozessoreinrichtung erlaubt. Gemäß einer besonders bevorzugten Ausführungsform des erfindungsgemäßen Verfahrens umfasst die Grafikmarkierung jedoch eine Mehrzahl oder Vielzahl geometrischer Elemente wie beispielsweise, jedoch keineswegs ausschließlich, Linien, Blöcke, Kreise oder Ellipsen, wobei jedes der geometrischen Elemente zumindest einen bekannten und festgelegten geometrischen Bezug gegenüber einem Fixpunkt der Grafikmarkierung besitzt.

Aufgrund des bekannten und festgelegten geometrischen Bezugs der einzelnen Bestandteile der Grafikmarkierung, beispielsweise anhand charakteristischer Abmessungen, Formgebungen und/oder Relativpositionen der einzelnen geometrischen Elemente der Grafikmarkierung zueinander, lässt sich die Genauigkeit der Kamerakalibrierung zwischen Bildraum und Objektraum wie auch die Genauigkeit der Feststellung von Position bzw. Lage der Grafikmarkierung im Objektraum weiter ganz erheblich steigern.

Dies hängt damit zusammen, dass jedes von der Prozessoreinrichtung zusätzlich erkannte geometrische Element der Grafikmarkierung aufgrund seiner bekannten Abmessungen bzw. aufgrund seiner bekannten Relativposition gegenüber einem ebenso bekannten Fixpunkt der Grafikmarkierung zur Genauigkeit der Lokalisierung der Grafikmarkierung im Bildraum und damit auch zur Verbesserung der Genauigkeit der Kamerakalibrierung beitragen kann. Ein weiterer vorteilhafter Effekt dieser Ausführungsform besteht darin, dass aufgrund der zusätzlichen geometrischen Elemente und der damit gegebenen charakteristischeren Gestalt der Grafikmarkierung eine schnellere und leichtere automatische Identifizierung und Lokalisierung der Grafikmarkierung innerhalb der von der Bildaufnahmekamera gelieferten Bilddatei möglich ist.

Dabei hat es sich gezeigt, dass sich bei geeigneter Gestaltung der Grafikmarkierung, insbesondere bei einer Ausführung der Grafikmarkierung mit einer Vielzahl geometrischer Elemente, beispielsweise mit einer Vielzahl (zusätzlicher) Liniensegmente, die Genauigkeit der Positionsermittlung der Grafikmarkierung im Bildraum und damit auch die Genauigkeit der Kamerakalibrierung und der Positionsermittlung der Grafikmarkierung im Objektraum nahezu beliebig steigern lässt. Dies führt insbesondere zu dem entscheidenden Vorteil, dass die physikalische Auflösung der Bildaufnahmekamera keinen limitierenden Faktor für die Genauigkeit der Positionsbestimmung der Grafikmarkierung mehr darstellt. Vielmehr lässt sich durch geeignete Gestaltung der Grafikmarkierung bzw. durch geeignete Anordnung der geometrischen Elemente einer Grafikmarkierung eine Genauigkeit der Positionsbestimmung erreichen, die bis weit in den Subpixelbereich der Bildaufnahmekamera hineinreicht bzw. weit über die physikalische Auflösung der Bildaufnahmekamera hinausgeht.

Dies bedeutet, dass sich das erfindungsgemäße Verfahren bei entsprechender Ausgestaltung der Grafikmarkierungen auch für anspruchsvollste Messaufgaben mit höchsten Genauigkeitsanforderungen eignet, wie sie beispielsweise, jedoch keineswegs ausschließlich, bei der Ermittlung von zahnmedizinisch oder zahntechnisch relevanten Positions- und Bewegungsdaten der Zahnkiefer auftreten. Anhand geeigneter Ausführung und Gestaltung der Grafikmarkierungen können dabei vergleichsweise preiswerte Bildaufnahmekameras verwendet werden, ohne dass deren begrenzte physikalische Auflösung bereits eine Grenze für die erzielbare MessGenauigkeit darstellen würde.

Gemäß einer weiteren Ausführungsform des erfindungsgemäßen Verfahrens sind anhand einer charakteristischen Anordnung der geometrischen Elemente der Grafikmarkierung relativ zueinander bzw. relativ zu einem Fixpunkt der Grafikmarkierung Informationseinheiten codiert. Auf diese Weise lässt sich ein Mehrfachnutzen der geometrischen Elemente der Grafikmarkierung erzielen, indem die geometrischen Elemente sowohl zur Erhöhung der Genauigkeit der Kamerakalibrierung und der Positionsbestimmung der Grafikmarkierung dienen als auch zur Informationsspeicherung herangezogen werden können. Beispielsweise lässt sich anhand geeigneter charakteristischer Anordnung einiger oder aller geomtrischer Elemente einer Grafikmarkierung - zur leichteren automatischen Zuordnung durch die Prozessoreinrichtung - eine Identifikationsnummer der jeweiligen Grafikmarkierung kodieren oder es können charakteristische Abmessungen der jeweiligen Grafikmarkierung auf diese Weise gespeichert werden.

Das erfindungsgemäße Verfahren lässt sich unabhängig davon verwirklichen, auf welche Weise die Grafikmarkierung mit dem zugehörigen Körperteil des Patienten verbunden ist. Gemäß einer Ausführungsform des erfindungsgemäßen Verfahrens ist in diesem Zusammenhang jedoch vorgesehen, dass die Grafikmarkierung mit einem Bereich der Oberfläche des Körperteils reibschlüssig oder haftschlüssig verbunden ist. Auf diese Weise lässt sich die Verbindung zwischen Grafikmarkierung und Körperteil besonders einfach realisieren, beispielsweise indem die Grafikmarkierung als auf Haut oder Kleidung anzuordnender Aufkleber ausgebildet wird oder beispielsweise indem die Grafikmarkierung mit einem strumpfartigen textilen Gebilde verbunden ist, das über das entsprechende Körperteil gestreift wird.

Mit diesem Hintergrund ist gemäß einer weiteren Ausführungsform des erfindungsgemäßen Verfahrens vorgesehen, dass eine Korrektur der ermittelten Messdaten anhand von gesammelten Erfahrungswerten über die Verschiebungen zwischen Hautoberfläche und Skelett erfolgt, die sich in Abhängigkeit der Relativposition der fraglichen Körperteile erfahrungsgemäß ergibt. Auf diese Weise lässt sich die Messgenauigkeit in den Fällen erhöhen, in welchen die Grafikmarkierungen der Einfachheit halber auf die Körperoberfläche aufgeklebt werden und somit nicht mit dem Skelett verbunden sind.

Gemäß einer weiteren, bevorzugten Ausführungsform des erfindungsgemäßen Verfahrens ist die zumindest eine Grafikmarkierung jedoch mit einem Teil des Skeletts verbunden. Auf diese Weise lässt sich eine besonders hohe Messgenauigkeit erzielen, da die durch Hautverschiebungen gegenüber dem Skelett verursachten Fehler auf diese Weise wegfallen. Besonders bevorzugt ist die Grafikmarkierung dabei mit zumindest einem Zahn des Oberkiefers bzw. des Unterkiefers verbunden. Somit lassen sich exakte Positions- bzw. Bewegungsdaten des menschlichen Schädels bzw. Kopfes ermitteln. Ferner können auf diese Weise die insbesondere für zahnmedizinische und zahntechnische Zwecke bedeutenden Relativpositions- und Relativbewegungsdaten von Oberkiefer und Unterkiefer mit der erforderlichen hohen Genauigkeit ermittelt werden.

Nach einer weiteren, besonders bevorzugten Ausführungsform des erfindungsgemäßen Verfahrens wird durch die Prozessoreinrichtung aus den ermittelten Daten bezüglich der Position und Bewegung des Unterkiefers bzw. bezüglich der Relativposition und Relativbewegung zwischen Oberkiefer und Unterkiefer automatisch die Lage der Kiefergelenkachse im dreidimensionalen Raum bzw. Lage der der Kondylen, also der Gelenkköpfe des Unterkiefers, ermittelt. Dies ist vorteilhaft insofern, als auf diese Weise die aufwändige manuelle Bestimmung der Lage der Kiefergelenkachse, die zudem fehlerbehaftet ist und eine Lagebestimmung mit nur mittlerer Genauigkeit erlaubt, entfällt und durch die automatische Lagebestimmung der Kiefergelenkachse ersetzt wird. Das Wissen um die Lage der Kiefergelenkachse ist in vielen Bereichen zahnmedizinischer Diagnose von großer Bedeutung, da diverse diagnostisch und zahntechnisch relevante Daten bezüglich der Kieferbewegung und der Okklusion häufig die Kiefergelenkachse sowie die Kondylen des Unterkiefers als Referenz verwenden.

Mit diesem Hintergrund ist es besonders bevorzugt vorgesehen, dass ermittelte Positions- oder Bewegungsdaten des Unterkiefers, bzw. Relativpositions- oder Relativbewegungsdaten zwischen Oberkiefer und Unterkiefer, mittels einer Koordinatentransformation durch die Prozessoreinrichtung automatisch auf einen auf der Kiefergelenkachse liegenden Punkt bezogen werden. Auf diese Weise lässt sich eine Vereinheitlichung der gewonnenen Daten und eine damit verbundene standardisierte Datenverwendbarkeit gewährleisten. Auch der Vergleich der auf diese Weise gewonnenen Daten über den Bewegungsablauf des Unterkiefers mit ähnlichen Daten, die jedoch auf herkömmliche Weise zum Beispiel mit einem aus dem Stand der Technik bekannten mechanischen oder elektronischen Pantographen erzeugt wurden, wird auf diese Weise erleichtert. Ein weiterer Vorteil dieser Ausführungsform liegt darin, dass die Grafikmarkierungen dank der automatischen Transformation der Messdaten auf einen auf der Kiefergelenkachse liegenden Punkt weitgehend frei und beliebig platziert werden können. Die Notwendigkeit, die Grafikmarkierungen exakt an ganz bestimmten Stellen der Kiefer zu platzieren, entfällt auf diese Weise, was dem unkomplizierten Messablauf und der zügigen Gewinnung der gewünschten Messwerte entscheidend entgegenkommt.

Gemäß weiterer bevorzugter Ausführungsformen des erfindungsgemäßen Verfahrens werden mittels der Prozessoreinrichtung aus den ermittelten Positions- bzw. Bewegungsdaten des Unterkiefers diagnostisch bzw. zahntechnisch relevante Daten, wie Kondylenachsenposition und -verlagerung, Kondylenbahnneigung und -verlauf, Bennettwinkel, Sideshift und dergleichen, automatisch abgeleitet; es wird aus den ermittelten Relativpositions- oder Relativbewegungsdaten der Kiefer sowie anhand von bekannten oder gemessenen Schädeldaten die Lage eines beliebigen Punktes des Unterkiefers ermittelt; oder es werden aus den ermittelten Positions- und Bewegungsdaten Kennwerte zur Einstellung zahntechnischer Artikulatoren abgeleitet.

Eine solche automatisierte Aufbereitung der am Kiefer gewonnenen Messdaten ist von großem Vorteil und von entscheidender Bedeutung sowohl bei der zuverlässigen zahnmedizinischen bzw. kieferorthopädischen Analyse und Diagnose als auch bei der kosteneffektiven, raschen und genauen Ausführung zahntechnischer Arbeiten durch den Zahntechniker, wie beispielsweise bei der Anfertigung von Zahnersatz mit Hilfe von Artikulatoren.

Die Erfindung lässt sich unabhängig davon verwirklichen, auf welche Weise die Grafikmarkierung mit dem Körperteil bzw. mit einem der Kiefer eines Patienten verbunden wird, solange eine einfache, zuverlässige und möglichst genaue Wiedergabe der Position bzw. Bewegung des entsprechenden Körperteils durch die Grafikmarkierung gegeben ist. Gemäß einer besonders bevorzugten Ausführungsform des erfindungsgemäßen Verfahrens wird hierzu jedoch eine Bissplatte mittels plastischer Bissmasse auf dem Zahnbogen des Unterkiefers angeordnet. Dabei ist die Bissplatte mit der zumindest einen Grafikmarkierung verbunden. Auf diese Weise ergibt sich eine einfache und leicht lösbare Verbindung zwischen dem Zahnbogen bzw. dem Unterkiefer und der Grafikmarkierung, die zudem aufgrund der Spielfreiheit der Verbindung eine höchstmögliche Genauigkeit bei der Positionsmessung und Bewegungsverfolgung zulässt.

Die Erfindung betrifft ferner eine Vorrichtung zur Aufzeichnung von Position, Lage oder Bewegung zumindest eines Bestandteils des Körpers eines Patienten oder eines ärztlichen Instruments. In für sich genommen zunächst bekannter Weise umfasst die Vorrichtung dabei zumindest eine mit dem Objektbestandteil - also mit dem zumindest einen Körperteil des Patienten oder mit dem zumindest einen Teil des ärztlichen Instrumentsverbindbare Grafikmarkierung sowie zumindest eine Bildaufnahmekamera, wobei die Bildaufnahmekamera so angeordnet und eingerichtet ist, dass sich mit der Bildaufnahmekamera zumindest eine die Grafikmarkierung umfassende Abbildung des Objektbestandteils erzeugen lässt. Die Aufzeichnungsvorrichtung umfasst ferner eine Prozessoreinrichtung zur automatisierten Verarbeitung von Bildinformationen, die in der durch die Bildaufnahmekamera erzeugten Abbildung enthalten sind.

Die Vorrichtung kann dabei zur Positions- oder Bewegungsverfolgung entweder des Körperteils des Patienten oder des ärztlichen Instruments ausgebildet sein, oder aber zur Positions- oder Bewegungsverfolgung sowohl des Körperteils des Patienten als auch des ärztlichen Instruments. Insbesondere im letzteren Fall können somit Aussagen über die Relativposition bzw. Relativbewegung zwischen dem Körperteil des Patienten und dem ärztlichen Instrument gemacht werden, die vor allem bei manuellen oder automatisierten chirurgischen Eingriffen von Bedeutung sind.

Erfindungsgemäß zeichnet sich die Aufzeichnungsvorrichtung dabei dadurch aus, dass zumindest ein geometrisches Größenbeschreibungsmerkmal der Grafikmarkierung bekannt sowie in einem Speicher der Prozessoreinrichtung aufnehmbar ist. Dabei ist die Prozessoreinrichtung zur Ermittlung von Position, Winkellage und/oder Bewegungsbahn der Grafikmarkierung relativ zur Bildaufnahmekamera anhand des bekannten Größenbeschreibungsmerkmals sowie anhand dessen Abmessungen im Bildraum eingerichtet.

Dies bedeutet mit anderen Worten, dass sich die erfindungsgemäße Vorrichtung insbesondere durch eine selbsttätige Kamerakalibrierung auszeichnet, wobei die Kamerakalibrierung anhand bekannter Informationen über die Abmessungen der Grafikmarkierung automatisch erfolgt.

Gegenüber dem Stand der Technik lässt sich dank der erfindungsgemäßen Vorrichtung die Lokalisierung bzw. Bewegungsverfolgung der Objektbestandteile, also der Teile eines Körpers bzw. eines medizinischen Instruments, - insbesondere aufgrund der selbsttätigen Kamerakalibrierung - somit zunächst einmal ganz erheblich vereinfachen. Ferner erlaubt die erfindungsgemäße Vorrichtung die Ermittlung sowohl der Position im dreidimensionalen Raum als auch ggf. die Feststellung der Winkellage des Objektbestandteils bzw. der mit dem Objektbestandteil verbundenen Grafikmarkierung relativ zur Bildaufnahmekamera. Dank der erfindungsgemäßen Vorrichtung lässt sich somit beispielsweise ein mit einer Grafikmarkierung versehener Körperteil eines Patienten, oder aber ein ärztliches Instrument, im dreidimensionalen Raum sowohl bezüglich seiner räumlichen Position als auch bezüglich seiner Winkellage lokalisieren.

Die Genauigkeit der Lokalisierung wird mit der erfindungsgemäßen Vorrichtung gegenüber dem Stand der Technik insbesondere bereits dadurch wesentlich verbessert, dass das erfindungsgemäße Verfahren nicht nur Aussagen über die Position der Grafikmarkierung gegenüber der optischen Achse der Bildaufnahmekamera erlaubt, sondern auch den Abstand zwischen Objekt bzw. Grafikmarkierung und Bildaufnahmekamera liefert.

Gemäß einer bevorzugten Ausführungsform zeichnet sich die erfindungsgemäße Aufzeichnungsvorrichtung dadurch aus, dass zumindest zwei Grafikmarkierungen vorgesehen sind, wobei für den Fall von zumindest zwei relativbeweglichen Körperteilen bzw. Objektbestandteilen jeweils zumindest eine Grafikmarkierung jedem der relativbeweglichen Objektbestandteile zugeordnet ist. Auf diese Weise kann auch die Relativposition der Objektbestandteile bzw. können auch die Relativbewegungen zwischen den Objektbestandteilen, beispielsweise zwischen zwei verschiedenen Körperteilen oder zwischen zwei verschiedenen Gliedern einer Extremität, mit hoher Zuverlässigkeit und Genauigkeit bestimmt werden.

Eine weitere Ausführungsform sieht vor, dass ein und demselben Objektbestandteil zumindest zwei Grafikmarkierungen zugeordnet sind. Damit lässt sich mit der Aufzeichnungsvorrichtung die Position des Objektbestandteils wie auch die Lage des Objektbestandteils im dreidimensionalen Raum mit besonders hoher Genauigkeit bestimmen. Dies gilt insbesondere dann, wenn die Prozessoreinrichtung so eingerichtet ist, dass die bekannte geometrische Relation - beispielsweise der bekannte Abstand zwischen den zwei Grafikmarkierungen - ebenfalls in die Auswertung durch die Prozessoreinrichtung mit einbezogen werden kann.

Gemäß weiterer Ausführungsformen der Aufzeichnungsvorrichtung sind zumindest zwei Bildaufnahmekameras vorgesehen. Dabei sind die Bildaufnahmekameras gemäß einer Ausführungsform so eingerichtet, dass sich ein und dieselbe Grafikmarkierung durch die zumindest zwei Bildaufnahmekameras aufnehmen lässt. Auf diese Weise wird die Zuverlässigkeit und Genauigkeit der' Positions- bzw. Lageermittlung im dreidimensionalen Objektraum weiter erhöht.

Gemäß einer weiteren Ausführungsform der erfindungsgemäßen Aufzeichnungsvorrichtung sind zumindest einem Objektbestandteil hingegen zumindest zwei Grafikmarkierungen zugeordnet, wobei die Grafikmarkierungen zur Anordnung in unterschiedlichen, vorzugsweise kartesischen bzw. orthogonalen, Raumrichtungen vorgesehen sind, und wobei für jede der Raumrichtungen eine eigene Bildaufnahmekamera vorgesehen ist.

Auf diese Weise lässt sich die Genauigkeit der Ermittlung der Position bzw. Winkellage oder die Genauigkeit der Aufzeichnung eines Bewegungsablaufs des Objektbestandteils im dreidimensionalen Raum noch weiter erhöhen.

Die Erfindung wird unabhängig davon verwirklicht, wie die zumindest eine verwendete Grafikmarkierung konkret ausgestaltet ist bzw. welche geometrischen Merkmale die Grafikmarkierung aufweist, solange ein ausreichender Kontrast vorhanden ist, der die Erfassung und automatische Auswertung der Aufnahmen aus der Bildaufnahmekamera durch die Prozessoreinrichtung erlaubt. Gemäß einer besonders bevorzugten Ausführungsform der Erfindung umfasst die Grafikmarkierung jedoch eine Mehrzahl oder Vielzahl geometrischer Elemente wie beispielsweise Blöcke, Kreise oder Ellipsen, wobei jedes der geometrischen Elemente zumindest einen bekannten und jeweils festgelegten geometrischen Bezug gegenüber einem Fixpunkt der Grafikmarkierung aufweist.

Aufgrund des bekannten und festgelegten geometrischen Bezugs der einzelnen Bestandteile der Grafikmarkierung - zum Beispiel anhand charakteristischer Abmessungen und/oder Relativpositionen der einzelnen geometrischen Elemente der Grafikmarkierung zueinander - lässt sich die Genauigkeit der Kamerakalibrierung zwischen Bildraum und Objektraum wie auch die Genauigkeit der Feststellung von Position bzw. Lage der Grafikmarkierung im Objektraum erheblich weiter steigern. Eine weitere vorteilhafte Eigenschaft dieser Ausführungsform der erfindungsgemäßen Vorrichtung besteht darin, dass aufgrund der zusätzlichen geometrischen Elemente eine schnellere und leichtere automatische Identifizierung und Lokalisierung der Grafikmarkierung in der von der Bildaufnahmekamera gelieferten Bilddatei erfolgen kann, da sich eine so ausgebildete Grafikmarkierung deutlicher und mit weniger Verwechslungsgefahr vom Hintergrund bzw. von anderen in der Bilddatei enthaltenen Bildelementen abhebt.

Dies bedeutet, dass die erfindungsgemäße Vorrichtung bei entsprechender Ausgestaltung der Grafikmarkierungen auch für anspruchsvolle Messaufgaben mit höchsten Genauigkeitsanforderungen eingesetzt werden kann, wie dies - beispielsweise - bei der kieferorthopädischen Diagnose bzw. bei der Ermittlung von zahnmedizinisch oder zahntechnisch relevanten Positions- und Bewegungsdaten der Zahnkiefer der Fall ist. Anhand einer geeigneten Ausführung und Gestaltung der Grafikmarkierungen können somit aber auch vergleichsweise preiswerte Bildaufnahmekameras verwendet werden, ohne dass deren begrenzte physikalische Auflösung eine Grenze für die erzielbare Messgenauigkeitbilden würde.

Gemäß einer weiteren Ausführungsform der erfindungsgemäßen Vorrichtung sind anhand einer charakteristischen Anordnung der geometrischen Elemente der Grafikmarkierung relativ zueinander bzw. relativ zu einem Fixpunkt der Grafikmarkierung Informationseinheiten codiert. Auf diese Weise wird ein Mehrfachnutzen der geometrischen Elemente der Grafikmarkierung erzielt, indem die geometrischen Elemente sowohl zur Erhöhung der Genauigkeit der Kamerakalibrierung und der Positionsbestimmung dienen als auch sich zur Informationsspeicherung heranziehen lassen. Beispielsweise kann mittels geeigneter charakteristischer Anordnung der geometrischen Elemente einer Grafikmarkierung - zur leichteren automatischen Identifikation durch die Prozessoreinrichtung - für jede Grafikmarkierung eine eigene Identifikationsnummer kodiert werden, oder es können charakteristische Abmessungen der jeweiligen Grafikmarkierung auf diese Weise im Muster der Grafikmarkierung selbst gespeichert werden.

Die Erfindung lässt sich unabhängig davon verwirklichen, auf welche Weise die Grafikmarkierung mit dem zugehörigen Objektbestandteil, also mit dem zugehörigen Körperteil des Patienten oder mit dem zugehörigen Teil des ärztlichen Instruments, verbunden wird. Gemäß einer Ausführungsform der Erfindung ist die Vorrichtung jedoch für eine reibschlüssige oder haftschlüssige Verbindung der Grafikmarkierung mit einem Bereich der Objektoberfläche eingerichtet. Auf diese Weise kann die Verbindung zwischen Grafikmarkierung und Objektbestandteil besonders einfach und rasch hergestellt werden, beispielsweise indem die Grafikmarkierung als Aufkleber ausgebildet wird, der auf Haut, Kleidung oder Instrument angeordnet werden kann, oder beispielsweise indem die Grafikmarkierung mit einem strumpfartigen textilen Gebilde verbunden ist, das sich über das entsprechende Körperteil ziehen lässt.

Gemäß einer weiteren, bevorzugten Ausführungsform der erfindungsgemäßen Aufzeichnungsvorrichtung ist die Vorrichtung zur Verbindung der Grafikmarkierung mit einem Teil des Skeletts eingerichtet. Auf diese Weise lässt sich eine besonders hohe Messgenauigkeit erzielen, insbesondere da durch Hautverschiebungen gegenüber dem Skelett verursachte Messfehler auf diese Weise wegfallen. Mit diesem Hintergrund weist die Vorrichtung gemäß einer weiteren Ausführungsform eine Befestigungsanordnung zur Verbindung der Grafikmarkierung mit zumindest einem Zahn des Oberkiefers oder des Unterkiefers auf. Auf diese Weise können mit der Aufzeichnungsvorrichtung exakte Positions- bzw. Bewegungsdaten des menschlichen Schädels bzw. Kopfes ermittelt werden. Ferner lassen sich so auch die insbesondere für zahnmedizinische und zahntechnische Zwecke bedeutenden Relativpositions- und Relativbewegungsdaten von Oberkiefer und Unterkiefer mit der erforderlichen hohen Genauigkeit ermitteln.

Die erfindungsgemäße Vorrichtung kann unabhängig davon eingesetzt werden, ob eine definierte Relation oder mechanische Fixierung zwischen der Vorrichtung und dem Körper des Patienten bzw. dem ärztlichen Instrument vorhanden ist oder nicht. So ist es mit der erfindungsgemäßen Vorrichtung insbesondere möglich, Relativbewegungen zwischen verschiedenen Objektbestandteilen mit hoher Genauigkeit zu ermitteln und zu verfolgen, ohne dass irgend eine definierte mechanische Verbindung zwischen der Aufzeichnungsvorrichtung und dem Verfolgungsobjekt bzw. Patienten vorhanden ist.

Gemäß einer bevorzugten Ausführungsform der Erfindung umfasst die Aufzeichnungsvorrichtung jedoch eine Einrichtung zur Fixierung der zumindest einen Bildaufnahmekamera relativ zu einem Teil des Körpers bzw. Skeletts des Patienten. Insbesondere für den Fall des Einsatzes der erfindungsgemäßen Aufzeichnungsvorrichtung im Bereich der zahnmedizinischen Analyse und Diagnose kann eine derartige Fixierung zwischen Bildaufnahmekamera und Patient beispielsweise in Form eines Gurtes, einer speziellen Kopfstütze oder einer elastischen Klammer erfolgen, wobei Gurt, Kopfstütze oder Klammer an den Schädel bzw. an den Kopf des Patienten angelegt werden.

Auf diese Weise wird der Kopf bzw. der Schädelknochen des Patienten und damit auch der Oberkiefer in eine definierte und vorübergehend unveränderliche Relativposition zur der mit Gurt, Kopfstütze oder Klammer verbundenen Aufzeichnungsvorrichtung bzw. Bildaufnahmekamera gebracht. Dies dient einerseits der Vereinfachung der Durchführung des Messvorgangs und andererseits der weiteren Erhöhung der Messgenauigkeit. Zudem kann somit auf die Anbringung von Grafikmarkierungen am Oberkiefer oder im Bereich des Schädels bzw. Kopfes des Patienten verzichtet werden.

Gemäß einer weiteren bevorzugten Ausführungsform ist die Aufzeichnungsvorrichtung mit einer Anzeigeeinheit verbindbar. Dabei sind Prozessoreinrichtung bzw. Anzeigeeinheit so eingerichtet, dass anhand der ermittelten Position, Lage und/oder Bewegungsbahn der zumindest einen Grafikmarkierung diagnostisch relevante Daten und Zusammenhänge, wie Kondylenachsenposition und -verlagerung, Kondylenbahnneigung und -verlauf, Bennettwinkel, Sideshift oder Posseltschema, automatisch ermittelt bzw. grafisch oder numerisch angezeigt werden können.

Die automatische Aufbereitung und Anzeige der am Kiefer gewonnenen Messdaten ist von entscheidendem Vorteil sowohl bei der zahnmedizinischen bzw. kieferorthopädischen Analyse und Diagnose als auch bei der Ausführung zahntechnischer Arbeiten - wie beispielsweise bei der Anfertigung von Zahnersatz mit Hilfe eines Artikulators - durch den Zahntechniker.

Mit diesem Hintergrund ist es gemäß einer weiteren Ausführungsform der Erfindung ferner vorgesehen, dass die Aufzeichnungsvorrichtung zur automatischen Ermittlung von charakteristischen Kenndaten für die unmittelbare Einstellung verschiedener Typen zahntechnischer Artikulatoren eingerichtet ist. Auf diese Weise lassen sich die mit der erfindungsgemäßen Aufzeichnungsvorrichtung ermittelten kieferorthopädisch bzw. zahnmedizinisch relevanten Daten mit geringem Aufwand zuverlässig auf den vom Zahntechniker jeweils verwendeten Artikulator übertragen.

Im Folgenden wird die Erfindung anhand einer lediglich ein Ausführungsbeispiel darstellenden Zeichnung näher erläutert.

Es zeigt die einzige:
- **Fig.**: in nicht maßstabsgetreuer, schematischer Darstellung eine Ausführungsform einer erfindungsgemäßen Vorrichtung in isometrischer Ansicht.

In der **Fig.** ist in höchst schematischer Weise eine Ausführungsform einer Aufzeichnungsvorrichtung gemäß der vorliegenden Erfindung dargestellt. Die in der Fig. dargestellte Aufzeichnungsvorrichtung ist zum Einsatz insbesondere in der zahnärztlichen Praxis vorgesehen und dient der sogenannten Unterkiefer-Registrierung bzw. der damit verbundenen Aufzeichnung der Bewegungsbahnen des Unterkiefers bei der Mundöffnung, bei Okklusionsbewegungen und dergleichen.

Man erkennt zunächst einmal den Kopf 1 des Patienten, der mittels einer elastischen, gepolsterten Klammer 2 relativ zu einem Sockel 3 der Aufzeichnungsvorrichtung fixiert ist. Anstelle der gepolsterten Klammer 2 kann beispielsweise auch eine spezielle Kopfstütze verwendet werden, wobei die Aufzeichnungsvorrichtung in diesem Fall vorzugsweise mit der Kopfstütze bzw. mit dem Kopfteil einer Behandlungsliege der zahnärztlichen Praxis verbunden ist.

An dem Sockel 3 der Aufzeichnungsvorrichtung ist außer der gepolsterten Klammer 2 zur Fixierung des Kopfes 1 des Patienten ein verstellbarer Kamerabügel 4 angeordnet. An den Kamerabügel 4 sind bei der dargestellten Ausführungsform drei lediglich höchst schematisch angedeutete Bildaufnahmekameras 5, 6, 7 angeordnet, deren Objektive 8 zunächst ungefähr auf den Patienten hin ausgerichtet sind. Vorteilhaft können hierzu handelsübliche und kostengünstige CCD-Kameras beispielsweise mit USB- oder Firewire-Interface verwendet werden. Die Bildaufnahme-kameras 5, 6, 7 sind variabel an den Kamerabügel 4 angebunden, so dass eine möglichst weiträumige Verstellbarkeit der Bildaufnahmekameras 5, 6, 7 relativ zum Sockel 3 der Aufzeichnungseinrichtung bzw. relativ zum Patientenkopf 1 gegeben ist.

Der Patient trägt im Mund eine (in der **Fig.** nicht sichtbare) Bissplatte, die mittels einer plastischen Bissmasse mit den Zähnen des Unterkiefers verbunden ist und damit sämtlichen Bewegungen des Unterkiefers folgt. Mit der Bissplatte ist eine Anzahl von Markierungsflächen 9 verbunden. Bezüglich des Mundes des Patienten in anteriorer Position sind die Markierungsflächen 9 auf einem Vierkantstab 10 angeordnet, wobei der Vierkantstab 10 wiederum mit der im Mund des Patienten angeordneten Bissplatte verbunden ist. Lateral bezüglich des Kopfes 1 des Patienten im ungefähren Bereich der Kiefergelenke befinden sich weitere Markierungsflächen 9, die im dargestellten Ausführungsbeispiel in Form der Oberflächen von Winkelprofilen 11 ausgebildet sind. Dabei sind die Winkelprofile 11 mit den lateralen Markierungsflächen und der die anterioren Markierungsflächen tragende Vierkantstab 10 mittels eines leichten, vom Vierkantstab 10 abnehmbaren Verbindungsbogens 12 gekoppelt.

Auf den Markierungsflächen 9 sowohl im anterioren 10 als auch im lateralen Bereich 11 sind jeweils eine Anzahl von Grafikmarkierungen bzw. Marker 13 angeordnet. Die Marker 13 sind im dargestellten Ausführungsbeispiel der Einfachheit und Darstellbarkeit halber als simple schwarze Kreise ausgebildet bzw. dargestellt.

Außer der gepolsterten Klammer 2 bzw. einer entsprechenden Kopfstütze und der Bissplatte mit den damit verbundenen Markierungsflächen 9 sind im Unterschied zum Stand der Technik keine weiteren mit dem Kopf 1 des Patienten zu verbindenden Vorrichtungen, Messbügel, Sensorhalterungen oder dergleichen erforderlich. Dies bringt ganz wesentliche Vorteile in Bezug auf die für den Bediener rasche und unkomplizierte sowie für den Patienten kaum belastende Durchführung der erfindungsgemäß ausgeführten Unterkiefer-Registrierung mit sich.

Zur Messung der Bewegungsbahnen des Unterkiefers des Patienten werden die Bildaufnahmekameras 5, 6, 7 durch entsprechende Verschiebung des Kamerabügels 4 bzw. durch Verschiebung der einzelnen Kameras 5, 6, 7 relativ zum Kamerabügel 4 so ausgerichtet, dass das Blickfeld jeder Kamera 5, 6, 7 eine Anzahl von Grafikmarkierungen bzw. Markern 13 erfasst. In diesem Zustand werden die von den Bildaufnahmekameras 5, 6, 7 erzeugten Bilddateien bzw. Pixelbilder in regelmäßigen Zeitintervallen durch die Prozessoreinrichtung 14 nach dem erfindungsgemäßen Verfahren ausgewertet.

Bei dem dargestellten Ausführungsbeispiel kann die Messung entweder kiefergelenksnah durch Aufzeichnung und Verfolgung der auf den Winkelprofilen 11 angeordneten, lateralen Markierungsflächen und Grafikmarkierungen erfolgen; oder aber es kann eine Messung mit Hilfe des frontalen Stifts bzw. Vierkantstabs 10 erfolgen, indem die Bildaufnahmekameras 5, 6, 7 auf die anterior angeordneten Markierungsflächen auf dem Vierkantstab 10 gerichtet werden. Bei der Messung mit frontalem Stift 10 wird somit der Verbindungsbogen 12 mit den kiefergelenksnahen, lateral bei 11 angeordneten Markierungsflächen und Grafikmarkierungen nicht benötigt und kann daher zusammen mit den Winkelprofilen 11 entfernt werden, was einen apparativ extrem einfachen und für den Patienten praktisch nicht belastenden Messaufbau lediglich mit Bissplatte und Vierkantstab 10 ergibt.

Die Messung erfolgt dabei jeweils in der Form, dass die Prozessoreinrichtung 14 mittels eines Bilderkennungsalgorithmus die von den Bildaufnahmekameras 5, 6, 7 gelieferten Bilddateien bzw. Pixelbilder auf die in den Pixelbildern jeweils enthaltenen Abbildungen der Grafikmarkierungen bzw. Marker 13 untersucht und anschließend die Position der in den Pixelbildern aufgefundenen Grafikmarkierungen bzw. Marker 13 im dreidimensionalen Raum anhand des erfindungsgemäßen Verfahrens automatisch bestimmt.

Da sich erfindungsgemäß die Absolutposition der Grafikmarkierungen im dreidimensionalen Raum relativ zu der jeweils zugeordneten Bildaufnahmekamera 5, 6, 7 bestimmen lässt und da das erfindungsgemäße Verfahren und die erfindungsgemäße Vorrichtung zudem eine Genauigkeit der Positionsverfolgung der Grafikmarkierungen erlauben, die bis weit in den Subpixelbereich der Bildaufnahmekameras 5, 6, 7 reicht, ermöglicht die dargestellte Vorrichtung die Registrierung bzw. Aufzeichnung der Bewegungen des Unterkiefers mit einer im Stand der Technik bisher praktisch unerreichten Genauigkeit.

Dies hängt zunächst einmal insbesondere damit zusammen, dass erfindungsgemäß eine selbsttätige Kalibrierung der Bildaufnahmekameras 5, 6, 7 erfolgt, indem die Prozessoreinrichtung 14 anhand der Abmessungen der Grafikmarkierungen bzw. Marker 13 auf dem von der jeweiligen Bildaufnahmekamera 5, 6, 7 gelieferten Pixelbild sowie anhand der bekannten Geometrie- bzw. anhand der realen Abmessungsdaten der Grafikmarkierungen im Objektraum den tatsächlichen Abstand zwischen jeweiliger Grafikmarkierung und zugehöriger Bildaufnahmekamera 5, 6, 7 bestimmt. Diese selbsttätige Kalibrierung der Bildaufnahmekameras 5, 6, 7 durch die Prozessoreinrichtung 14 kann zudem fortlaufend - beispielsweise sogar im Rahmen der Verarbeitung jedes einzelnen Einzelbilds - erneuert werden, so dass zu jedem Zeitpunkt der genaue Abstand zwischen Bildaufnahmekamera 5, 6, 7 und Grafikmarkierung bzw. Marker 13 bekannt ist.

Anhand des dergestalt ermittelten, bekannten Abstands zwischen Grafikmarkierung bzw. Marker 13 und zugehöriger Bildaufnahmekamera 5, 6, 7 kann jedoch unter Berücksichtigung der Abbildungseigenschaften des Kameraobjektivs die Absolutposition der Grafikmarkierung relativ zur Bildaufnahmekamera 5, 6, 7 bestimmt werden. Dies ist einer der entscheidenden Unterschiede zum Stand der Technik, wo entweder gänzlich auf absolute Messungen verzichtet wird, womit primär qualitative Aussagen über die aufgezeichneten Bewegungsbahnen möglich sind, oder bei dem eine aufwändige manuelle Kalibrierung der verwendeten Kameras oder Sensorsysteme erforderlich ist.

Ein weiterer entscheidender Faktor zu der mit der dargestellten Aufzeichnungsvorrichtung erreichten extrem hohen Genauigkeit der Lokalisierung und Verfolgung der Grafikmarkierungen bzw. Marker 13 liegt darin, dass anhand geeigneter Gestaltung der Grafikmarkierungen die Genauigkeit der Positionsverfolgung fast beliebig gesteigert werden kann. Bei dem dargestellten Ausführungsbeispiel wird hierzu neben dem bekannten Durchmesser der einzelnen Marker 13 beispielsweise auch noch der bekannte Abstand zwischen jeweils zwei auf derselben Markierungsfläche 9 angeordneten Markern 13 herangezogen. Bereits durch die zusätzliche Heranziehung des bekannten Abstands zwischen den paarweise angeordneten Markern 9 kann die Genauigkeit der Kamerakalibrierung gesteigert und die Präzision der Lokalisierung und Verfolgung der Grafikmarkierungen um weitere Größenordnungen bis in den Mikrometerbereich hinein verbessert werden.

Dies hängt insbesondere damit zusammen, dass jedes bekannte geometrische Merkmal und jede bekannte Abmessung der Grafikmarkierung die Genauigkeit der Lokalisierung und Verfolgung der Grafikmarkierung innerhalb der durch die Bildaufnahmekamera 5, 6, 7 gelieferten Bilddatei erhöht. Denn durch jedes zusätzliche geometrische Merkmal der Grafikmarkierung wird der mittels entsprechender Bilderkennungsalgorithmen (beispielsweise Houghtransformation) auswertbare Informationsgehalt der Abbildung der Grafikmarkierung gesteigert; und es steht zudem eine jeweils entsprechend vergrößerte Anzahl von Bildinformationen, beispielsweise Bildpixeln, zur Verfügung, die somit zur noch genaueren Lokalisierung der Grafikmarkierung herangezogen werden kann.

Zur Aufzeichnung des gesamten Bewegungsablaufs des Unterkiefers würden bei dem dargestellten Ausführungsbeispiel auch bereits die im anterioren Mundbereich auf dem Stift 10 angeordneten Markierungsflächen 9 mit einer dort angeordneten Grafikmarkierung sowie prinzipiell bereits eine einzige der drei vorliegend verwendeten Bildaufnahmekameras 5, 6, 7 genügen. Die im lateralen Kopfbereich in der Region der Kiefergelenke bei 11 anordenbaren zusätzlichen Markierungsflächen bzw. Grafikmarkierungen sowie die vorliegend verwendete Anzahl von drei Bildaufnahmekameras 5, 6, 7 steht somit im Dienst vor allem der Erhöhung der Genauigkeit bei der Bewegungsverfolgung des Unterkiefers. Dabei dient die im oberen Bereich des Kamerabügels angeordnete Bildaufnahmekamera 7 vor allem der genauen Aufzeichnung der seitlichen Bewegungen, also des Sideshift-Verlaufs, des Unterkiefers, der bei Mundöffnungsbewegungen in aller Regel auftritt.

Der ferner im vorderen Bereich der Aufzeichnungsvorrichtung erkennbare Verbindungsbügel 15 dient der einfachen Einstellung der Rotationsposition der beiden lateralen Kameras 5 und 6, wobei der Verbindungsbügel 15 nach erfolgter Einstellung der Kameras 5 und 6 abgezogen werden kann, um freien Zugang zum Patienten 1 zu ermöglichen. Die Einstellung der Rotationsposition der lateralen Kameras 5 und 6 dient dazu, die erhaltenen Messdaten bzw. Bewegungskurven des Unterkiefers in Relation zur tatsächlichen Position des Schädels bzw. Kopfes 1 des Patienten zu setzen.

Die Kameraeinstellung der lateralen Kameras 5 und 6 erfolgt dabei dergestalt, dass die lateral angeordneten Bildaufnahmekameras 5 und 6 mit Hilfe des Verbindungsbügels 15 so lange um die Kameraachse verdreht bzw. verschwenkt werden, bis der Verbindungsbügel 15 und damit eine jeweils definierte Horizontalachse der Kamera 5, 6 parallel zur Frankfurter Horizontalen, also zur gedachten Verbindungslinie zwischen Porion (Oberrand des äußeren Gehörgangs) und Orbita (knöcherne Augengrube), ausgerichtet ist.

Außer der Prozessoreinrichtung 14 weist die dargestellte Aufzeichnungsbzw. Registriereinrichtung ferner auf - bzw. ist damit verbunden - eine Ausgabeeinrichtung 16, beispielsweise einen Bildschirm und/oder einen Drucker, ein Bedienfeld 17, beispielsweise eine Tastatur, eine (externe) Speichereinrichtung 18, beispielsweise einen USB-Speicherstick, sowie eine Datenverbindung zu einem externen Rechner 19, beispielsweise zu einem Personalcomputer, insbesondere um dort weitere Detailauswertungen der ermittelten Messdaten vornehmen zu können.

Im Ergebnis wird somit deutlich, dass mit der Erfindung die Möglichkeiten zur Aufzeichnung bzw. Verfolgung von Position, Lage oder Bewegung von Teilen des Körpers eines Patienten bzw. von ärztlichen Instrumenten entscheidend erweitert und dabei gleichzeitig vereinfacht werden. Die Erfindung ermöglicht dabei sowohl eine hochgradige Automatisierung als auch eine hohe Messgenauigkeit bei der Positionsermittlung und Bewegungsverfolgung, wobei gleichzeitig der apparative Aufwand entscheidend reduziert und die Rückwirkung auf das zu messende Objekt oder Körperteil bzw. auf den Patienten minimiert ist.

Die Erfindung liefert damit einen entscheidenden Beitrag zur Verbesserung der Aufzeichnung von Absolut- und Relativpositionen bzw. -bewegungen insbesondere in Bereichen, wie beispielsweise in der manuellen oder automatisierten Chirurgie oder in der zahnmedizinischen Kieferregistrierung, in denen höchste Messgenauigkeit und einfache Anwendbarkeit zentrale Anwendungskriterien darstellen.

## Patentansprüche

1. Verfahren zur Aufzeichnung von Position, Lage oder Bewegung zumindest eines Teils eines in einem Objektraum angeordneten Körpers eines Patienten mittels elektronischer Bilderkennung anhand zumindest einer Bildaufnahmekamera (5, 6, 7), das Verfahren aufweisend die nachfolgenden Verfahrensschritte:
a) Verbindung zumindest einer Grafikmarkierung mit zumindest einem Körperteil, wobei zumindest ein geometrisches Größenbeschreibungsmerkmal der Grafikmarkierung bekannt ist;
b) Erzeugung einer Abbildung zumindest des die Grafikmarkierung enthaltenden Bereichs des Körperteils durch die Bildaufnahmekamera;
c) Übernahme der Abbildung als Bilddatei in eine elektronische Prozessoreinrichtung (14);
d) im Bildraum der Bilddatei Lokalisierung des in der Bilddatei enthaltenen Abbilds der Grafikmarkierung mittels Anwendung einer automatisierten Bilderkennungsprozedur auf die Bilddatei;
e) Ermittlung des Größenbeschreibungsmerkmals der Grafikmarkierung im Bildraum;
f) Ermittlung von Position und/oder Lage der Grafikmarkierung im Objektraum relativ zur Bildaufnahmekamera (5, 6, 7) anhand des Größenbeschreibungsmerkmals im Bildraum und im Objektraum;
g) Übernahme der ermittelten Daten bezüglich der Position der Grafikmarkierung im Objektraum in einen Datenspeicher.

2. Verfahren nach Anspruch 1,
**dadurch gekennzeichnet,**
**dass** zur Aufzeichnung, einer Ortsveränderung der Grafikmarkierung bzw. eines Bewegungsablaufs des Körperteils die Verfahrensschritte b) bis g) wiederholt werden.

3. Verfahren nach Anspruch 1 oder 2,
**dadurch gekennzeichnet,**
**dass** jeweils zumindest eine Grafikmarkierung mit zumindest zwei relativbeweglichen Körperteilen verbunden ist.

4. Verfahren nach einem der Ansprüche 1 bis 3,
**dadurch gekennzeichnet,**
**dass** mit dem zumindest einen Körperteil zumindest zwei Grafikmarkierungen verbunden sind.

5. Verfahren nach einem der Ansprüche 1 bis 4,
**dadurch gekennzeichnet,**
**dass** zumindest zwei Bildaufnahmekameras (5, 6, 7) zur Aufnahme von Abbildungen der zumindest einen Grafikmarkierung vorgesehen sind.

6. Verfahren nach einem der Ansprüche 1 bis 5,
**dadurch gekennzeichnet,**
**dass** mit zumindest einem Körperteil zumindest zwei unterschiedlichen Raumrichtungen zugeordnete Grafikmarkierungen verbunden sind, wobei für jede der Raumrichtungen eine eigene Bildaufnahmekamera (5, 6, 7) vorgesehen ist.

7. Verfahren nach Anspruch 6,
**dadurch gekennzeichnet,**
**dass** die unterschiedlichen Raumrichtungen einem kartesischen Koordinatensystem entsprechen.

8. Verfahren nach einem der Ansprüche 1 bis 7,
**dadurch gekennzeichnet,**
**dass** die ermittelten Positionsdaten zu orthopädischen oder zahntechnischen Zwecken bzw. zur Registrierung von Unterkieferbewegungen verwendet werden.

9. Verfahren nach einem der Ansprüche 1 bis 8,
**dadurch gekennzeichnet,**
**dass** die Grafikmarkierung eine Mehrzahl oder Vielzahl geometrischer Elemente (13) aufweist, wobei jedes der geometrischen Elemente (13) zumindest einen bekannten, festgelegten geometrischen Bezug zu einem Fixpunkt der Grafikmarkierung besitzt.

10. Verfahren nach Anspruch 9,
**dadurch gekennzeichnet,**
**dass** mittels einer charakteristischen Anordnung der geometrischen Elemente (13) relativ zueinander und/oder relativ zu einem Fixpunkt der Grafikmarkierung Informationseinheiten codiert sind.

11. Verfahren nach einem der Ansprüche 1 bis 10,
**dadurch gekennzeichnet,**
**dass** die Grafikmarkierung mit einem Bereich der Oberfläche des Körperteils reibschlüssig oder haftschlüssig verbunden ist.

12. Verfahren nach Anspruch 3 und Anspruch 11,
**dadurch gekennzeichnet,**
**dass** in Abhängigkeit der ermittelten Relativposition der zumindest zwei Körperteile eine Korrektur der ermittelten Messdaten anhand von Erfahrungswerten über die in Abhängigkeit der Relativposition der Körperteile auftretenden Verschiebungen zwischen Hautoberfläche und Skelett erfolgt.

13. Verfahren nach einem der Ansprüche 1 bis 12,
**dadurch gekennzeichnet,**
**dass** die Grafikmarkierung mit einem Teil des Skeletts verbunden ist.

14. Verfahren nach einem der Ansprüche 1 bis 13,
**dadurch gekennzeichnet,**
**dass** die Grafikmarkierung mit zumindest einem Zahn des Ober- oder Unterkiefers verbunden ist.

15. Verfahren nach Anspruch 14,
**dadurch gekennzeichnet,**
**dass** durch die Prozessoreinrichtung (14) aus den ermittelten Positions- oder Bewegungsdaten des Unterkiefers automatisch die Lage der Kiefergelenkachse bzw. der Kondylen ermittelt wird.

16. Verfahren nach Anspruch 14 oder 15,
**dadurch gekennzeichnet,**
**dass** ermittelte Positions- oder Bewegungsdaten des Unterkiefers mittels einer Koordinatentransformation auf einen auf der Kiefergelenkachse liegenden Punkt bezogen werden.

17. Verfahren nach einem der Ansprüche 14 bis 16,
**dadurch gekennzeichnet,**
**dass** mittels Prozessoreinrichtung (14) aus den ermittelten Positions- oder Bewegungsdaten zahnmedizinisch bzw. zahntechnisch relevante Daten, wie Kondylenachsenposition und -verlagerung, Kondylenbahnneigung und -verlauf, Bennettwinkel, Sideshift u. dgl., automatisch abgeleitet werden.

18. Verfahren nach einem der Ansprüche 14 bis 17,
**dadurch gekennzeichnet,**
**dass** mittels Prozessoreinrichtung (14) aus den ermittelten Positions- oder Bewegungsdaten sowie anhand von Schädeldaten die Lage eines beliebigen Punktes des Unterkiefers ermittelt wird.

19. Verfahren nach einem der Ansprüche 14 bis 18,
**dadurch gekennzeichnet,**
**dass** mittels Prozessoreinrichtung (14) aus den ermittelten Positions- oder Bewegungsdaten Kenndaten zur Einstellung zahntechnischer Artikulatoren abgeleitet werden.

20. Verfahren nach einem der Ansprüche 14 bis 19,
**dadurch gekennzeichnet,**
**dass** eine Bissplatte mittels Bissmasse auf dem Zahnbogen des Unterkiefers angeordnet wird, wobei die Bissplatte mit zumindest einer Grafikmarkierung verbunden ist.

21. Vorrichtung zur Aufzeichnung von Position, Lage oder Bewegung zumindest eines Bestandteils des Körpers eines Patienten oder eines ärztlichen Instruments, die Vorrichtung umfassend zumindest eine mit dem Objektbestandteil verbindbare Grafikmarkierung und zumindest eine Bildaufnahmekamera (5, 6, 7), wobei die Bildaufnahmekamera (5, 6, 7) zur Erzeugung zumindest einer die Grafikmarkierung umfassenden Abbildung des Objektbestandteils eingerichtet ist, die Vorrichtung ferner umfassend eine Prozessoreinrichtung (14) zur automatisierten Verarbeitung von in der Abbildung enthaltenen Bildinformationen,
**dadurch gekennzeichnet,**
**dass** zumindest ein geometrisches Größenbeschreibungsmerkmal der Grafikmarkierung bekannt und in einen Speicher der Prozessoreinrichtung (14) aufnehmbar ist, wobei die Prozessoreinrichtung (14) zur Ermittlung von Position, Lage und/oder Bewegungsbahn der Grafikmarkierung relativ zur Bildaufnahmekamera (5, 6, 7), anhand des bekannten Größenbeschreibungsmerkmals sowie anhand dessen Abmessungen im Bildraum, eingerichtet ist.

22. Vorrichtung nach Anspruch 21,
**gekennzeichnet durch**
zumindest zwei Grafikmarkierungen, wobei jede der Grafikmarkierungen jeweils einem von zumindest zwei relativbeweglichen Objektbestandteilen zugeordnet ist.

23. Vorrichtung nach Anspruch 21 oder 22,
**gekennzeichnet durch**
zumindest zwei Grafikmarkierungen, wobei die zumindest zwei Grafikmarkierungen demselben Objektbestandteil zugeordnet sind.

24. Vorrichtung nach einem der Ansprüche 21 bis 23,
**gekennzeichnet durch**
zumindest zwei Bildaufnahmekameras (5, 6, 7) zur Aufnahme von Abbildungen der zumindest einen Grafikmarkierung.

25. Vorrichtung nach einem der Ansprüche 21 bis 24,
**dadurch gekennzeichnet,**
**dass** zumindest einem Objektbestandteil zumindest zwei in unterschiedlichen Raumrichtungen angeordnete Grafikmarkierungen zugeordnet sind, wobei die Vorrichtung eine eigene Bildaufnahmekamera (5, 6, 7) für jede Raumrichtung umfasst.

26. Vorrichtung nach Anspruch 25,
**dadurch gekennzeichnet,**
**dass** die unterschiedlichen Raumrichtungen einem kartesischen Koordinatensystem entsprechen.

27. Vorrichtung nach einem der Ansprüche 21 bis 26,
**dadurch gekennzeichnet,**
**dass** die Grafikmarkierung eine Mehrzahl oder Vielzahl geometrischer Elemente (13) aufweist, wobei jedes der geometrischen Elemente (13) im Objektraum zumindest einen bekannten, festgelegten geometrischen Bezug zu einem Fixpunkt der Grafikmarkierung besitzt.

28. Vorrichtung nach Anspruch 27,
**dadurch gekennzeichnet,**
**dass** mittels einer charakteristischen Anordnung der geometrischen Elemente (13) relativ zueinander und/oder relativ zu einem Fixpunkt der Grafikmarkierung Informationseinheiten codiert sind.

29. Vorrichtung nach einem der Ansprüche 21 bis 28,
**dadurch gekennzeichnet,**
**dass** die Vorrichtung für eine reibschlüssige oder haftschlüssige Verbindung der Grafikmarkierung mit einem Bereich der Körperoberfläche eingerichtet ist.

30. Vorrichtung nach einem der Ansprüche 21 bis 29,
**dadurch gekennzeichnet,**
**dass** die Vorrichtung zur Verbindung der Grafikmarkierung mit einem Teil des Skeletts eingerichtet ist.

31. Vorrichtung nach einem der Ansprüche 21 bis 30,
**dadurch gekennzeichnet,**
**dass** die Vorrichtung eine Befestigungsanordnung (10, 11, 12) zur Verbindung der Grafikmarkierung mit zumindest einem Zahn des Ober- oder Unterkiefers aufweist.

32. Vorrichtung nach einem der Ansprüche 21 bis 31,
**dadurch gekennzeichnet,**
**dass** die Vorrichtung eine Einrichtung (2) zur Fixierung der Bildaufnahmekamera relativ zu einem Teil des Körpers bzw. Skeletts umfasst.

33. Vörrichtung nach Anspruch 31 oder 32,
**dadurch gekennzeichnet,**
**dass** die Prozessoreinrichtung (14) mit einer Anzeigeeinheit (16) verbindbar ist, wobei Prozessoreinrichtung (14) bzw. Anzeigeeinheit (16) anhand der ermittelten Position, Lage und/oder Bewegungsbahn der zumindest einen Grafikmarkierung zur automatischen Ermittlung bzw. zur grafischen oder numerischen Anzeige zahnmedizinisch relevanter Zusammenhänge, wie Kondylenachsenposition und -verlagerung, Kondylenbahnneigung und -verlauf, Bennettwinkel, Sideshift, Posseltschema und dergleichen, eingerichtet sind.

34. Vorrichtung nach einem der Ansprüche 31 bis 33,
**dadurch gekennzeichnet,**
**dass** die Vorrichtung zur automatischen Ermittlung von Kenndaten zur Einstellung zahntechnischer Artikulatoren eingerichtet ist.

## Claims

1. A method for recording a position, orientation or movement of at least a part of a patient's body situated in an object space by means of electronic image recognition using at least one imaging camera (5, 6, 7), the method comprising the following method steps:
a) connecting at least one graphic marker to at least one body part, wherein at least one geometric variable description feature of the graphic marker is known;
b) creating an image of at least the area of the body part containing the marker using the imaging camera;
c) transferring the image to an electronic processing device (14) as a picture file;
d) in the image space of the image file, locating the copy of the graphic marker contained in the image file by applying an automated image recognition procedure to the image file;
e) calculating the variable description feature of the graphic marker in the image space;
f) calculating the position and / or orientation of the graphic marker in the object space relative to the imaging camera (5, 6, 7) on the basis of the variable description feature in the image space and in the object space;
g) transferring the calculated data relating to the position of the graphic marker in the object space to a data memory.

2. The method according to claim 1,
***characterized in that***
method steps b) to g) are repeated for recording a change of location of the graphic marker and/or a movement sequence of the body part.

3. The method according to claim 1 or 2,
***characterized in that***
each of the at least one graphic markers is connected to at least two relatively movable body parts.

4. The method according to any of claims 1 to 3,
***characterized in that***
at least two graphic markers are connected to the at least one body part.

5. The method according to any of claims 1 to 4,
***characterized in that***
at least two imaging cameras (5, 6, 7) are provided for capturing images of the at least one graphic marker.

6. The method according to any of claims 1 to 5,
***characterized in that***
at least two graphic markers allocated to different spatial directions are connected to at least one body part, wherein a separate imaging camera (5, 6, 7) is provided for each of said spatial directions.

7. The method according to claim 6,
***characterized in that***
the different spatial directions correspond to a Cartesian coordinate system.

8. The method according to any of claims 1 to 7,
***characterized in that***
the calculated position data is used for orthopaedic or dental purposes, and/or for registering movements of the lower jaw.

9. The method according to any of claims 1 to 8,
***characterized in that***
the graphic marker comprises a plurality or a multiplicity of geometric elements (13), wherein each of said geometric elements (13) has at least one known, fixed geometrical reference to a fixed point of the graphic marker.

10. The method according to claim 9,
***characterized in that***
information units are encoded by means of a characteristic arrangement of the geometric elements (13) relative to each other and/or relative to a fixed point of the graphic marker.

11. The method according to any of claims 1 to 10,
***characterized in that***
the graphic marker is engaged in a frictional or adhesive lock with an area of the surface of the body part.

12. The method according to claim 3 and claim 11,
***characterized in that***
depending on the calculated relative positions of the at least two body parts, the calculated measurement data is adjusted on the basis of empirical values for the shifts occurring between the skin surface and the skeleton depending on the relative positions of the body parts.

13. The method according to any of claims 1 to 12,
***characterized in that***
the graphic marker is connected to a part of the skeleton.

14. The method according to any of claims 1 to 13,
***characterized in that***
the graphic marker is connected to at least one tooth in the upper or lower jaw.

15. The method according to claim 14,
***characterized in that***
the orientation of the temporomandibular joint axis and the condyles is calculated automatically by the processor device (14) from the calculated positional or movement data for the lower jaw.

16. The method according to claim 14 or 15,
***characterized in that***
calculated positional or movement data for the lower jaw is related to a point lying on the temporomandibular joint axis by means of a coordinate transformation.

17. The method according to any of claims 14 to 16,
***characterized in that***
medically and technically pertinent dental data such as the position and displacement of the condylar axis, condylar track inclination and progression, Bennett angle, side shift and the like, is derived automatically from the calculated positional or movement data by means of the processor device (14).

18. The method according to any of claims 14 to 17,
***characterized in that***
the position of any point of the lower jaw is calculated from the calculated positional or movement data and based on skull data by means of the processor device (14).

19. The method according to any of claims 14 to 18,
***characterized in that***
characteristic data for the setting of dental articulators is derived from the calculated positional or movement data by means of the processor device (14).

20. The method according to any of claims 14 to 19,
***characterized in that***
a bite plate is arranged on the dental arch of the lower jaw ground using bite mass, wherein the bite plate is connected to at least one graphic marker.

21. A device for recording the position, orientation or movement of at least one component of a patient's body or of a dental instrument, the device comprising at least one graphic marker that is connectable to the object component and at least one imaging camera (5, 6, 7), wherein the imaging camera (5, 6, 7) is arranged to generate at least one image of the object component that includes the graphic marker, the device further comprises a processor device (14) for the automated processing of picture data contained in the image
***characterized in that***
at least one geometric variable description feature of the graphic marker is known and can be stored in a memory of the processor device (14), wherein the processor device (14) is set up to calculate the position, orientation and/or movement path of the graphic marker relative to the imaging camera (5, 6, 7) on the basis of the known variable description feature and with reference to the dimensions thereof in the image space.

22. The device according to claim 21,
***characterized by***
at least two graphic markers, wherein each of the graphic markers is assigned to one each of at least two relatively movable object components.

23. The device according to claim 21 or 22,
***characterized in that***
at least two graphic markers, wherein the at least two markers are assigned to the same object component.

24. The device according to any of claims 21 to 23,
***characterized in that***
at least two imaging cameras (5, 6, 7) for recording images of the at least one graphic marker.

25. The device according to any of claims 21 to 24,
***characterized in that***
at least two graphic markers arranged in different spatial directions are assigned to at least one object component, wherein the device comprises a dedicated imaging camera (5, 6, 7) for each spatial direction.

26. The device according to claim 25,
***characterized in that***
the different spatial directions correspond to a Cartesian coordinate system.

27. The device according to any of claims 21 to 26,
***characterized in that***
the graphic marker comprises a plurality or a multiplicity of geometric elements (13), wherein each of said geometric elements (13) has at least one known, fixed geometrical reference to a fixed point of the graphic marker in the object space.

28. The device according to claim 27,
***characterized in that***
information units are encoded using a characteristic arrangement of the geometric elements (13) relative to each other and/or relative to a fixed point of the graphic marker.

29. The device according to any of claims 21 to 28,
***characterized in that***
the device is configured to enable a frictional or adhesive lock of the graphic marker with a region of the body surface.

30. The device according to any of claims 21 to 29,
***characterized in that***
the device is configured to connect the graphic marker to a part of the skeleton.

31. The device according to any of claims 21 to 30,
***characterized in that***
the device comprises a fastening arrangement (10, 11, 12) for connecting the graphic marker to at least one tooth in the upper or lower jaw.

32. The device according to any of claims 21 to 31,
***characterized in that***
the device comprises an apparatus (2) for fixing the imaging camera relative to a part of the body or skeleton.

33. The device according to claim 31 of 32,
***characterized in that***
the processor device (14) can be connected to a display unit (16), wherein said processor device (14) and display unit (16) are set up using the calculated position, orientation and/or movement track of the at least one graphic marker for the automatic calculation and/or graphical or digital display of dentally relevant relationships, such as the position and displacement of the condylar axis, condylar track inclination and progression, Bennett angle, side shift Posselt diagram and the like.

34. The device according to any of claims 31 to 33,
***characterized in that***
the device is configured for the automatic calculation of characteristic data for setting dental articulators.

## Revendications

1. Procédé d'enregistrement de la position, de la situation ou du mouvement au moins d'une partie du corps d'un patient, disposé dans un espace objet, au moyen d'une reconnaissance d'image électronique à l'aide d'au moins une caméra de prise de vues (5, 6, 7), le procédé présentant les étapes de procédé suivantes consistant à :
a) relier au moins un repère graphique à au moins une partie du corps, dans lequel au moins un renseignement de grandeur géométrique du repère graphique est connu ;
b) à l'aide de la caméra de prise de vues, générer une image au moins de la zone de la partie du corps qui contient le repère graphique ;
c) reporter l'image sous forme de fichier d'image dans un équipement à processeur électronique (14) ;
d) dans l'espace image du fichier d'image, localiser l'image du repère graphique, contenue dans le fichier d'image, en appliquant au fichier d'image une procédure de reconnaissance d'image automatique ;
e) détecter le renseignement de grandeur du repère graphique dans l'espace image ;
f) détecter la position et/ou la situation du repère graphique dans l'espace objet par rapport à la caméra de prise de vues (5, 6, 7) à l'aide du renseignement de grandeur dans l'espace image et dans l'espace objet ;
g) reporter dans une mémoire de données les données détectées concernant la position du repère graphique dans l'espace objet.

2. Procédé selon la revendication 1,
***caractérisé en ce que***
pour enregistrer un changement d'emplacement du repère graphique ou d'une séquence de mouvement de la partie du corps, les étapes de procédé b) à g) sont répétées.

3. Procédé selon la revendication 1 ou 2,
***caractérisé en ce que***
respectivement au moins un repère graphique est relié à au moins deux parties du corps à mouvement relatif.

4. Procédé selon l'une quelconque des revendications 1 à 3,
***caractérisé en ce qu'***
au moins deux repères graphiques sont reliés à ladite au moins une partie du corps.

5. Procédé selon l'une quelconque des revendications 1 à 4,
***caractérisé en ce qu'***
au moins deux caméras de prise de vues (5, 6, 7) sont prévues pour enregistrer des images dudit au moins un repère graphique.

6. Procédé selon l'une quelconque des revendications 1 à 5,
***caractérisé en ce que***
des repères graphiques reliés à au moins deux directions spatiales différentes sont reliés à au moins une partie du corps, une caméra de prise de vues (5, 6, 7) spécifique étant prévue pour chacune des directions spatiales.

7. Procédé selon la revendication 6,
***caractérisé en ce que***
les différentes directions spatiales correspondent à un système de coordonnées cartésiennes.

8. Procédé selon l'une quelconque des revendications 1 à 7,
***caractérisé en ce que***
les données de position détectées sont utilisées à des fins orthopédiques ou prothétiques ou pour enregistrer des mouvements de la mâchoire inférieure.

9. Procédé selon l'une quelconque des revendications 1 à 8,
***caractérisé en ce que***
le repère graphique présente une multitude ou une pluralité d'éléments géométriques (13), chacun desdits éléments géométriques (13) possédant au moins une relation géométrique définie connue par rapport à un point fixe du repère graphique.

10. Procédé selon la revendication 9,
***caractérisé en ce que***
des unités d'information sont codées au moyen d'une disposition caractéristique des éléments géométriques (13) les uns par rapport aux autres et/ou par rapport à un point fixe du repère graphique.

11. Procédé selon l'une quelconque des revendications 1 à 10,
***caractérisé en ce que***
le repère graphique est relié à une zone de la surface de la partie du corps par friction ou par adhérence.

12. Procédé selon les revendications 3 et 11,
***caractérisé en ce qu'***
en fonction de la position relative détectée desdites au moins deux parties du corps, une correction des données de mesure détectées est effectuée à l'aide de valeurs empiriques concernant les décalages entre la surface de la peau et le squelette survenant en fonction de la position relative des parties du corps.

13. Procédé selon l'une quelconque des revendications 1 à 12,
***caractérisé en ce que***
le repère graphique est relié à une partie du squelette.

14. Procédé selon l'une quelconque des revendications 1 à 13,
***caractérisé en ce que***
le repère graphique est relié à au moins une dent de la mâchoire supérieure ou inférieure.

15. Procédé selon la revendication 14,
***caractérisé en ce qu'***
partir des données de position ou de mouvement détectées de la mâchoire inférieure, l'équipement à processeur (14) détecte automatiquement la situation de l'axe de l'articulation temporo-mandibulaire ou des condyles.

16. Procédé selon la revendication 14 ou 15,
***caractérisé en ce que***
des données de position ou de mouvement détectées de la mâchoire inférieure sont mises en relation avec un point situé sur l'axe de l'articulation temporo-mandibulaire au moyen d'une transformation de coordonnées.

17. Procédé selon l'une quelconque des revendications 14 à 16,
***caractérisé en ce qu'***
à partir des données de position ou de mouvement détectées, des données significatives sur le plan dentaire ou prothétique, telles que la position et le décalage de l'axe condylien, la pente et la courbe de la trajectoire condylienne, l'angle de Bennett, le déplacement latéral ou similaires, sont dérivées automatiquement au moyen de l'équipement à processeur (14).

18. Procédé selon l'une quelconque des revendications 14 à 17,
***caractérisé en ce qu'***
à partir des données de position ou de mouvement détectées, ainsi qu'à l'aide de données du crâne, la situation d'un point quelconque de la mâchoire inférieure est détectée au moyen de l'équipement à processeur (14).

19. Procédé selon l'une quelconque des revendications 14 à 18,
***caractérisé en ce qu'***
à partir des données de position ou de mouvement, des données caractéristiques pour régler des articulateurs de prothèse sont dérivées au moyen de l'équipement à processeur (14).

20. Procédé selon l'une quelconque des revendications 14 à 19,
***caractérisé en ce qu'***
une plaque d'occlusion est disposée sur l'arcade dentaire de la mâchoire inférieure au moyen d'une cire d'occlusion, la plaque d'occlusion étant reliée à au moins un repère graphique.

21. Dispositif d'enregistrement de la position, de la situation ou du mouvement au moins d'un élément du corps d'un patient ou d'un instrument médical, ledit dispositif comprenant au moins un repère graphique pouvant être relié à l'élément d'objet et au moins une caméra de prise de vues (5, 6, 7), dans lequel la caméra de prise de vues (5, 6, 7) est aménagée pour générer au moins une image de l'élément d'objet comprenant le repère graphique, ledit dispositif comprenant en outre un équipement à processeur (14) pour un traitement automatique des informations d'image contenues dans l'image,
***caractérisé en ce qu'***
au moins un renseignement de grandeur géométrique du repère graphique est connu et peut être enregistré dans une mémoire de l'équipement à processeur (14), dans lequel l'équipement à processeur (14) est aménagé pour détecter la position, la situation et/ou la trajectoire de mouvement du repère graphique par rapport à la caméra de prise de vues (5, 6, 7), à l'aide du renseignement de grandeur connu ainsi qu'à l'aide de ses dimensions dans l'espace image.

22. Dispositif selon la revendication 21,
***caractérisé par***
au moins deux repères graphiques, dans lequel chacun des repères graphiques est attribué respectivement à l'un parmi au moins deux éléments d'objet à mouvement relatif.

23. Dispositif selon la revendication 21 ou 22,
***caractérisé par***
au moins deux repères graphiques, dans lequel lesdits au moins deux repères graphiques sont attribués au même élément d'objet.

24. Dispositif selon l'une quelconque des revendications 21 à 23,
***caractérisé par***
au moins deux caméras de prise de vues (5, 6, 7) pour enregistrer des images dudit au moins un repère graphique.

25. Dispositif selon l'une quelconque des revendications 21 à 24,
***caractérisé en ce qu'***
au moins deux repères graphiques disposés dans différentes directions spatiales sont attribués à au moins un élément d'objet, dans lequel le dispositif comprend une caméra de prise de vues (5, 6, 7) spécifique pour chaque direction spatiale.

26. Dispositif selon la revendication 25,
***caractérisé en ce que***
les différentes directions spatiales correspondent à un système de coordonnées cartésiennes.

27. Dispositif selon l'une quelconque des revendications 21 à 26,
***caractérisé en ce que***
le repère graphique présente une multitude ou une pluralité d'éléments géométriques (13), dans lequel chacun des éléments géométriques (13) possède dans l'espace objet au moins une référence géométrique définie connue à un point fixe du repère graphique.

28. Dispositif selon la revendication 27,
***caractérisé en ce que***
des unités d'information sont codées au moyen d'une disposition caractéristique des éléments géométriques (13) les uns par rapport aux autres et/ou par rapport à un point fixe du repère graphique.

29. Dispositif selon l'une quelconque des revendications 21 à 28,
***caractérisé en ce que***
le dispositif est aménagé pour relier par friction ou par adhérence des repères graphiques à une zone de la surface du corps.

30. Dispositif selon l'une quelconque des revendications 21 à 29,
***caractérisé en ce que***
le dispositif est aménagé pour relier le repère graphique à une partie du squelette.

31. Dispositif selon l'une quelconque des revendications 21 à 30,
***caractérisé en ce que***
le dispositif présente un agencement de fixation (10, 11, 12) pour relier le repère graphique à au moins une dent de la mâchoire supérieure ou inférieure.

32. Dispositif selon l'une quelconque des revendications 21 à 31,
***caractérisé en ce que***
ce que le dispositif comprend un équipement (2) pour fixer la caméra de prise de vues par rapport à une partie du corps ou du squelette.

33. Dispositif selon la revendication 31 ou 32,
***caractérisé en ce que***
l'équipement à processeur (14) peut être relié à une unité d'affichage (16), dans lequel l'équipement à processeur (14) ou l'unité d'affichage (16) est aménagé(e) pour déterminer automatiquement ou pour afficher de manière graphique ou numérique, à partir des données de position ou de mouvement détectées, des relations significatives sur le plan dentaire ou prothétique, telles que la position et le décalage de l'axe condylien, la pente et la courbe de la trajectoire condylienne, l'angle de Bennett, le déplacement latéral ou similaires.

34. Dispositif selon l'une quelconque des revendications 31 à 33,
***caractérisé en ce que***
le dispositif est aménagé pour détecter automatiquement des données caractéristiques permettant de régler des articulateurs de prothèse.
